# EUROPÄISCHE PATENTSCHRIFT

(11) **EP 1 395 231 B1**
(45) Veröffentlichungstag und Bekanntmachung des Hinweises auf die Patenterteilung: **01.06.2005**
(21) Anmeldenummer: 02732724.6
(22) Anmeldetag: 17.05.2002
(51) Int. Cl.: A61K 7/04, A61K 7/043, A61K 31/17, A61K 7/48

(54) **ZUBEREITUNG ZUR ENTFERNUNG VON ABNORMEN KERATINMATERIAL**
PREPARATION FOR THE REMOVAL OF ABNORMAL KERATIN MATERIAL
PREPARATION DESTINEE A ELIMINER LA MATIERE KERATINIQUE ANORMALE

(30) Priorität: 30.05.2001 DE 10126501
(43) Veröffentlichungstag der Anmeldung: 10.03.2004
(73) Patentinhaber: Aventis Pharma Deutschland GmbH, 65929 Frankfurt am Main (DE)
(72) Erfinder: ULBRICHT, Horst, 63599 Biebergemünd (DE); POOTH, Rainer, 63303 Dreieich-Götzenhain (DE); SHUSTER, Samuel, Framlingham IP13 9BN (GB)
(86) Internationale Anmeldenummer: PCT/EP2002/005469
(87) Internationale Veröffentlichungsnummer: WO 2002/098380

(56) Entgegenhaltungen:
- EP-A- 0 298 271
- DE-A- 3 810 897
- US-A- 4 721 724
- US-A- 5 993 790
- R.J. HAY; D.T. ROBERTS; V.R. DOHERTY; M.D. RICHARDSON; G. MIDGLEY: "The topical treatment of onychomycosis using a new combined urea/imidazole preparation" CLINICAL EXPERIMENTAL DERMATOLOGY, Nr. 13, 1988, Seiten 164-167, XP001098064 in der Anmeldung erwähnt

## Beschreibung

Die vorliegende Erfindung betrifft eine Zubereitung enthaltend Harnstoff, einen hydrophilen Filmbildner sowie Wasser oder ein Alkohol/Wasser - Gemisch und deren Verwendung zur Entfernung von abnormen Keratinmaterial, wie es beispielsweise bei Onychomykosen, Nagelpsoriasis oder Warzen zu beobachten ist. Hinsichtlich des abnormen Keratinmaterials ist histologisch eine Hyperparakeratose erkennbar, die sich in einem abnorm veränderten Schichtaufbau der Haut oder des Nagels darstellt. Darüberhinaus kann durch diese Erfindung die physiologische Barrierefunktion beispielsweise von brüchigen Nägeln via Hydratation regeneriert werden.

Harnstoff wird seit Jahrzehnten in der dermatologischen Praxis angewandt. Cremes oder Lotionen sind bekannt. Harnstoff verändert die Struktur und die Eigenschaften des Keratins der Homschicht und der Nägel. Er hat eine wasserbindende Wirkung in der Homschicht in Abhängigkeit vom Träger sowie eine proliferationshemmende Wirkung auf die Epidermis. Harnstoff spaltet Disulfidbindungen und Wasserstoffbrücken-bindungen. Dadurch wird das tote keratinisierte Material aufgelockert und kann anschließend mechanisch abgelöst werden.
Für das Ablösen oder Auflösen veränderter, insbesondere pilzbefallener Nägel gibt es in Deutschland eine Creme mit 20% Harnstoff (Onychomal®) sowie eine Salbe, die außer 40% Harnstoff auch das Antimykotikum Bifonazol® (1%) enthält und zusammen mit wasserfesten Pflastern, einer Ausdrückhilfe und einem Nagelschaber in einer gemeinsamen Verpackung vertrieben wird (Mycospor® Nagelset). Diese Präparate sind seit über 10 Jahren im Handel (Bang DS, Lee YD, Whang KK, Lee SN). Therapeutic trial of ointment base including urea and antifungal agent as the treatment of onychomycosis. Ann Dermatol 1991; 3: 32-6; Hay RJ, Roberts DT, Doherty VR, Richardson MD, Midgley G. The topical treatment of onychomycosis using a new combined urea/imidazole preparation. Clin Exper Dermatol 1988; 13: 164-167).
Ferner ist auch ein Nagellack bekannt, enthaltend einen hydrophoben Filmbildner, ein Antimykotikum und Harnstoff, der zur Behandlung von Onychomykosen eingesetzt wird (US 5,346,692). In US 5,993,790 wird ein wässriger Nagellack beschrieben, der 85 - 95 Gew-% eines in Wasser gelösten Lackes und 0,5 - 10 Gew-% Harnstoff enthält. Dieser Lack dient zur Auflockerung von Nägeln, die durch einen Pilzbefall verändert wurden. Nachteilig für die Anwendung der bekannten Cremepäparate sind häufig auftretende Mazerationen und entzündliche Veränderungen der umgebenden Haut. Darüberhinaus erfordern die halbfesten Zubereitungen einen Verband an den betroffenen Stellen, um ein Abwischen zu verhindern, sowie einen Schutz des umliegenden Gewebes z. B. durch Abdecken mit Zinkpaste. Ein durchschlagender Erfolg blieb den bekannten Behandlungsmethoden versagt, weil die Behandlung - beispielsweise wegen der störenden und unschön aussehenden Pflaster an Zehen und Fingern und der täglich erforderlichen Maßnahmen - von den Patienten vielfach aus kosmetischen und Zeitgründen nicht durchgehalten wird. Der Zeitaufwand für das bisher übliche Verfahren ist verhältnismäßig hoch, und die Akzeptanz begrenzt oder die Compliance ist schnell erschöpft, wenn beispielsweise mehr als 3 bis 5 Nägel behandelt werden müssen.

Zur Ablösung von verhomten Hautarealen wie bei Warzen werden üblicherweise Salicylsäurezubereitungen in Form von halbfesten Zubereitungen wie Salicylvaseline (etwa 20 % - 60 %) oder Pflastern (Guttaplast® ) benutzt. Auch hier gelten in analoger Weise die Nachteile der halbfesten Zubereitungen.

Die Erfindung bezweckt durch die Bereitstellung einer Zubereitung, enthaltend einen hydrophilen Filmbildner, Harnstoff, Wasser und/oder ein Alkohol/Wasser - Gemisch, die genannten Nachteile zu verbessern.

Die erfindungsgemäße Zubereitung ist eine wässrige oder wässrig-alkoholische Lösung, worin der hydrophile Filmbildner und Harnstoff gelöst oder gegebenenfalls suspendiert sind. Vorteilhaft ist eine Lösung. Die Zubereitung bildet nach dem Auftragen auf das abnorme Keratinmaterial wie Warzen oder Fingernagel schnell einen haftenden, wischund abriebfesten Film, aus dem Harnstoff in das abnorme Keratin eindringt und dessen Ablösung unterstützt. Zusätzliche Abdeckungen mit Pflastern, das Auftragen eines speziellen Schutzfilmes für die den Zielort umgebenden Hautflächen sowie das tägliche Baden sind nicht erforderlich. Die erfindungsgemäße Zubereitung verhindert unerwünschte lokal auftretende Fällungsreaktionen des Harnstoffs auf dem zu behandelnden Keratinmaterial, die zu unansehnlichen Veränderungen oder zu möglichen Beeinträchtigungen der lokalen Bioverfügbarkeit führen. Die erfindungsgemäße Zubereitung ermöglicht vielmehr eine gleichmäßige Verteilung des Harnstoffs auf dem Keratinmaterial durch die erfindungsgemäße Zusammensetzung und dessen galenischen Eigenschaften.

Im Unterschied zu den im Stand der Technik bekannten Produkten bietet die Erfindung daher ein deutlich verbessertes Drug-Targeting wie die fokussierte Applikation am Zielorgan oder Zielort mit vermindertem Risiko für das benachbarte Gewebe sowie eine verbesserte Anwenderfreundlichkeit (Handling) bei der Applikation der erfindungsgemäßen Zubereitung.

Die erfindungsgemäße Zubereitung betrifft daher eine Formulierung, enthaltend
a) Harnstoff, in einer Menge von 41 bis 69 Gewichtsprozent, bezogen auf die nichtflüchtigen Bestandteile der Zubereitungen,
b) einen hydrophilen Filmbildner und
c) Wasser oder ein Alkohol-Wasser-Gemisch.

Die Zubereitung kann auch noch weitere flüchtige und nicht flüchtige Bestandteile enthalten, solange die Menge des Harnstoffs, bezogen auf die nichtflüchtigen Bestandteile der Zubereitungen, nicht über- oder unterschritten wird.

Die Mengen an Harnstoff sind jeweils bezogen auf die nichtflüchtigen Bestandteile der erfindungsgemäßen Zubereitung und sind bevorzugt von 45 Gewichts-% bis 65 Gewichts-%, insbesondere bevorzugt von 46 Gewichts-% bis 63 Gewichts-%, ferner bevorzugt von 55 Gewichts-% bis 63 Gewichts-%.

Als hydrophile Filmbildner kommen beispielsweise Acryl- / Methacrylsäureester-Copolymere, Polyvinylpyrrolidone, Polyvinylalkohole, Vinylacetat / Vinylpyrrolidon-Copolymere, Vinylacetat / Crotonsäure-Copolymere, Methylvinylether / MaleinsäureCopolymere, Polyester, Polyesteramide, Carboxymethylcellulose, Hydroxyethylcellulose. Hydroxypropylcellulose und Hydroxypropylmethylcellulose oder eine Mischung der genannten Filmbildner in Frage. Besonders geeignet sind Polyvinylpyrrolidone.

Die hydrophilen Filmbildner werden in Mengen von 30 Gewichts-% bis 60 Gewichts-%, bezogen auf die nichtflüchtigen Bestandteile, eingesetzt. Die Menge der hydrophilen Filmbildner hängt von der Menge des Harnstoffs ab und ergänzt sich in Abhängigkeit von der Harnstoffmenge und weiteren gegebenenfalls vorhandenen nichtflüchtigen Hilfsstoffen zu 100 %.

In den wässrig-alkoholischen Lösungen werden Alkohole beispielsweise (C₁- C₆)-Alkohole wie Methanol, Ethanol, Propanol, Isopropanol (2-Propanol), Butanol, Pentanol Hexanol oder Mischungen derselben eingesetzt. Bevorzugt werden Ethanol, n-Propanol oder 2-Propanol eingesetzt. In den wässrig-alkoholischen Lösungen beträgt das Verhältnis von Alkohol zu Wasser von 9 zu 1 bis 1 zu 9, bevorzugt sind 2 Teile Alkohol auf 3 Teile Wasser.

Als weitere Hilfsmittel sind Weichmacher wie Glycerintriacetat oder 1,2-Propylenglycol, und Mittel zur Einstellung des pH-Wertes der Zubereitungen, zum Beispiel Milchsäure oder Zitronensäure, geeignet. Bevorzugt wird Milchsäure in einer Menge von 0,5 Gewichts-% bis 5 Gewichts-% eingesetzt wird, bezogen auf das Gewicht der gesamten Zubereitung.

Die erfindungsgemäßen Zubereitungen können weiterhin in Kosmetika gebräuchliche Zusätze enthalten wie Weichmacher auf Phthalat-, Glyceryltriacetat- oder Campherbasis, Farbstoffe oder Farbpigmente, Perlglanzmittel, Sulfonamidharze, Sedimentationsverzögerer, Silikate, Riechstoffe, Netzmittel wie Natriumdioctylsulfosuccinat, Lanolinderivate, Lichtschutzmittel wie 2-Hydroxy-4-methoxybenzo-phenon oder antibakteriell wirksame Substanzen. Gefärbte oder pigmentierte Nagellacke haben beispielsweise den Vorteil, dass die erfindungsgemäße Zubereitung dem Schönheitsempfinden des Patienten angepaßt werden kann und die zwischenzeitlich bestehenden Nagelveränderungen für Dritte nicht unmittelbar sichtbar sind.

Die Herstellung der erfindungsgemäßen Zubereitung erfolgt durch Einbringen von Harnstoff und hydrophilen Filmbildner in Wasser/Alkohol und anschließenden Mischen. Bevorzugt werden wässrig-alkoholische Lösungen hergestellt, worin der Harnstoff in einer Menge von 15 % bis 35 % gelöst vorliegt, bezogen auf das Gewicht der gesamten Lösung. Die Menge an hydrophilen Filmbildner beträgt dann von etwa 15 % bis,etwa 35 %, jeweils bezogen auf das Gewicht der gesamten Lösung. Die Menge der hydrophilen Filmbildner hängt von der Menge des Harnstoffs ab und ergänzt sich in Abhängigkeit von der Harnstoffmenge und weiteren gegebenenfalls vorhandenen nichtflüchtigen Hilfsstoffen zu jeweils 100 %. Der Anteil von Wasser oder des wässrig-alkoholischen Gemisches beträgt von 30 % bis 60 %, bevorzugt von 35 % bis 55 %, jeweils bezogen auf das Gewicht der gesamten Lösung.

Die erfindungsgemäße Zubereitung wird bevorzugt als Lösung auf die zu behandelnden Keratinmaterialien aufgebracht. Sie trocknet schnell ein und bildet schnell einen haftenden, wisch- und abriebfesten Film. Die Aufbringung der Lösung erfolgt beispielsweise mit einem Pinsel.

Die Erfindung betrifft ferner die Verwendung der erfindungsgemäßen Zubereitung zur Herstellung eines Arzneimittels zur Ablösung von abnormen Keratinmaterial.

Unter dem Begriff "abnormen Keratinmaterial" wird Keratinmaterial bei Menschen und Tieren verstanden wie Warzen, Schwielen, Hornhaut oder Fuß- und Fingernägel, die durch einen Pilzbefall oder Psoriaserkrankung verändert wurde. Hinsichtlich des abnormen Keratinmaterials ist histologisch eine Hyperparakeratose erkennbar, die sich in einem abnorm veränderten Schichtaufbau der Haut oder des Nagels darstellt.

Die Erfindung betrifft auch die Verwendung einer Zubereitung, enthaltend
a) Harnstoff, in einer Menge von 25 Gewichtsprozent bis 35 Gewichtsprozent, bezogen auf das gesamte Gewicht der Zubereitung,
b) einen hydrophilen Filmbildner in einer Menge von 15 Gewichts-% bis 20 Gewichts-%, bezogen auf das gesamte Gewicht der Zubereitung und
c) Wasser oder ein Alkohol-Wasser-Gemisch.
zur Herstellung eines Arzneimittels zur Ablösung von abnormem Keratinmaterial.

Vorteilhaft sind Mischungen von 25 % bis 35 % Harnstoff mit 15 % bis 20 % hydrophilen Filmbildner, weil sie eine kürzere Trockenzeit aufweisen als Formulierungen mit einem höheren oder niedrigeren Gehalt an hydrophilen Filmbildner.

In der erfindungsgemäßen Verwendungen können die gleichen Alkohole wie bei der erfindungsgemäßen Zubereitung eingesetzt werden. Die Menge an Wasser und/oder Alkohol ist analog zu der erfindungsgemäßen Zubereitung. Die einsetzbaren hydrophilen Filmbildner entsprechen den genannten Filmbildnern für die erfindungsgemäße Zubereitung. Ferner können bei der erfindungsgemäßen Verwendung noch weitere Hilfsstoffe oder Zusätze wie bei der erfindungsgemäßen Zubereitung eingesetzt werden.

Die Erfindung betrifft auch die Verwendung einer wässrigen Lösung, enthaltend Harnstoff in einer Menge von 15 % bis 35 %, bevorzugt von 25 % bis 33 %, bezogen auf das Gewicht der gesamten Lösung, und einen hydrophilen Filmbildner in einer Menge von etwa 15 % bis etwa 35 %, bevorzugt von 17 % bis 25 %, jeweils bezogen auf das Gewicht der gesamten Lösung,
zur Herstellung eines Arzneimittels zur Behandlung von abnormem Keratinmaterial.

Das Ablösen des abnormen Keratinmaterials erfolgt durch Auftragung der Zubereitung und eine entsprechend lange Einwirkung der getrockneten Zubereitung auf dem zu behandelnden Keratinmaterial und anschließender mechanischer Entfernung des abnormen Keratinmaterials.

Die Erfindung betrifft ferner die Verwendung der erfindungsgemäßen Zubereitung zur Hydratisierung von brüchigen Fuß- oder Fingernägeln.

Die vorliegende Erfindung wird durch die folgenden Beispiele näher erläutert, jedoch nicht auf diese beschränkt. Soweit nichts anderes vermerkt, sind die Mengenangaben auf das Gewicht bezogen.

### Beispiel 1

Eine erfindungsgemäße Zubereitung weist folgende Zusammensetzung auf:

| | |
|---|---|
| Harnstoff | 30 % |
| Polyvinylpyrrolidon (Molekulargewicht etwa 11 500) | 20 % |
| Demineralisiertes Wasser | 50 % |

### Beispiel 2

Eine erfindungsgemäße Zubereitung weist folgende Zusammensetzung auf:

| | |
|---|---|
| Harnstoff | 30 % |
| Polyvinylpyrrolidon (Molekulargewicht etwa 11 500) | 20 % |
| Ethanol | 20 % |
| Demineralisiertes Wasser | 30 % |

### Beispiel 3

Eine erfindungsgemäße Zubereitung weist folgende Zusammensetzung auf:

| | |
|---|---|
| Harnstoff | 30 % |
| Polyvinylpyrrolidon (Molekulargewicht etwa 11 500) | 20 % |
| Propanol-2 | 20 % |
| Milchsäure | 1 % |
| Demineralisiertes Wasser | 29 % |

### Beispiel 4

Eine erfindungsgemäße Zubereitung weist folgende Zusammensetzung auf:

| | |
|---|---|
| Harnstoff | 30 % |
| Polyvinylpyrrolidon (Molekulargewicht etwa 11 500) | 20 % |
| Milchsäure | 1 % |
| Demineralisiertes Wasser | 49 % |

### Beispiel 5

Eine erfindungsgemäße Zubereitung weist folgende Zusammensetzung auf:

| | |
|---|---|
| Harnstoff | 30 % |
| Polyvinylpyrrolidon (Molekulargewicht etwa 15 000) | 20 % |
| Propanol-2 | 20 % |
| Demineralisiertes Wasser | 30 % |

### Beispiel 6

Eine erfindungsgemäße Zubereitung weist folgende Zusammensetzung auf:

| | |
|---|---|
| Harnstoff | 30 % |
| Polyvinylpyrrolidon (Molekulargewicht etwa 11 500) | 20 % |
| Cremophor EL | 1 % |
| Milchsäure | 1 % |
| Demineralisiertes Wasser | 48 % |

### Beispiel 7

### Wirksamkeitsprüfung

2 erkrankte Patienten wurden mit der Zubereitung gemäß Beispiel 3 an den Zehennägeln behandelt.

Die erfindungsgemäße Zubereitung gemäß Beispiel 3 wurde zweimal täglich vor dem Schlafengehen gezielt mit einem Pinsel auf die befallenen Nägel aufgetragen. Der nach dem Auftragen auf den Nägeln entstandene harnstoffhaltige Film war wisch- und wasserfest. Ein besonderer Schutz der die Nägel umgebenden Hautflächen sowie das Anbringen von Pflasterverbänden waren daher nicht erforderlich. Aufgrund des hohen Wassergehaltes der Zubereitungen wurden die befallenen Zehennägel nicht zusätzlich gebadet.

### Ergebnis:

Nach etwa 6 Tagen Behandlung konnten die befallenen Nagelareale und die subungualen Gewebstrümmer leicht mit einem Schaber entfernt werden. Die starke Nagelbrüchigkeit war verschwunden und die starke Hyperkeratose hatte sich zu einem mittleren Schweregrad gebessert.
Nach etwa 6 Wochen Behandlung zeigte der zweite Patient, dass seine starke Nagelbrüchigkeit verschwunden und die starke Hyperkeratose nicht mehr vorhanden war, i.e. guter Therapieerfolg.

Beide Patienten zeigten einen guten Behandlungserfolg. Die Verträglichkeit der erfindungsgemäßen Zubereitung war sehr gut. Beide Patienten waren mit der Handhabbarkeit beim Auftragen der Zubereitung sehr zufrieden.

## Patentansprüche

1. Zubereitung, **dadurch gekennzeichnet, dass** sie
a) Harnstoff, in einer Menge von 41 Gewichtsprozent bis 69 Gewichtsprozent, bezogen auf die nichtflüchtigen Bestandteile der Zubereitung,
b) einen hydrophilen Filmbildner und
c) Wasser oder ein Alkohol-Wasser-Gemisch, enthält.

2. Zubereitung nach Anspruch 1, **dadurch gekennzeichnet, dass** Harnstoff in einer Menge von 41 Gewichtsprozent bis 69 Gewichtsprozent, und/oder der hydrophile Filmbildner in einer Menge von 29 Gewichts-% bis 59 Gewichts-% enthalten ist, jeweils bezogen auf die nichtflüchtigen Bestandteile der Zubereitung.

3. Zubereitung nach Anspruch 2, **dadurch gekennzeichnet, dass** Harnstoff in einer Menge von 45 Gewichts-% bis 65 Gewichts-%, bevorzugt von 46 Gewichts-% bis 63 Gewichts-% enthalten ist, jeweils bezogen auf die nichtflüchtigen Bestandteile der Zubereitung.

4. Zubereitung nach Anspruch 3, **dadurch gekennzeichnet, dass** Harnstoff in einer Menge von 55 Gewichts-% bis 63 Gewichts-% enthalten ist, jeweils bezogen auf die nichtflüchtigen Bestandteile der Zubereitung.

5. Zubereitung nach einem oder mehreren der Ansprüche 1 bis 4, **dadurch gekennzeichnet, dass** als hydrophiler Filmbildner eine Verbindung aus der Gruppe Acryl-/Methacrylsäureester-Copolymere, Polyvinylpyrrolidone, Polyvinylalkohole, Vinylacetat /Vinylpyrrolidon- Copolymere, Vinylacetat / Crotonsäure-Copolymere, Methylvinylether /Maleinsäure- Copolymere, Polyester, Polyesteramide, Carboxymethylcellulose, Hydroxyethylcellulose. Hydroxypropylcellulose und Hydroxypropylmethylcellulose oder eine Mischung derselben eingesetzt wird.

6. Zubereitung nach Anspruch 5, **dadurch gekennzeichnet, dass** Polyvinylpyrrolidone als hydrophiler Filmbildner eingesetzt werden.

7. Zubereitung nach einem oder mehreren der Ansprüche 1 bis 6, **dadurch gekennzeichnet, dass** in der wässrig-alkoholischen Lösung ein Alkohol aus der Gruppe Methanol, Ethanol, Propanol, Isopropanol, Butanol, Pentanol und Hexanol oder Mischungen derselben eingesetzt wird.

8. Zubereitung nach Anspruch 7, **dadurch gekennzeichnet, dass** Ethanol, n-Propanol oder Isopropanol als Alkohol eingesetzt wird.

9. Zubereitung nach einem oder mehreren der Ansprüche 1 bis 8, **dadurch gekennzeichnet, dass** das Verhältnis von Alkohol zu Wasser von 9 zu 1 bis 1 zu 9 beträgt, bevorzugt sind 2 Teile Alkohol auf 3 Teile Wasser enthalten.

10. Zubereitung nach einem oder mehreren der Ansprüche 1 bis 9, **dadurch gekennzeichnet, dass** Milchsäure in einer Menge von 0,5 Gewichts-% bis 5 Gewichts-% eingesetzt wird, bezogen auf das Gewicht der gesamten Zubereitung.

11. Verwendung der Zubereitung nach einem oder mehreren der Ansprüche 1 bis 10, zur Herstellung eines Arzneimittels zur Ablösung von abnormen Keratinmaterial.

12. Verwendung nach Anspruch 11, **dadurch gekennzeichnet, dass** das abnorme Keratinmaterial aus der Gruppe Warzen, Schwielen, Hornhaut oder Fuß- und Fingernägel, wobei die Fuß- oder Fingernägel durch einen Pilzbefall oder Psoriaserkrankung verändert wurden, ausgewählt wird.

13. Verwendung einer Zubereitung, enthaltend
a) Harnstoff, in einer Menge von 25 Gewichtsprozent bis 35 Gewichtsprozent, bezogen auf das gesamte Gewicht der Zubereitung,
b) einen hydrophilen Filmbildner, der in einer Menge von 15 Gewichts-% bis 20 Gewichts-%, bezogen auf das gesamte Gewicht der Zubereitung, und
c) Wasser oder ein Alkohol-Wasser-Gemisch.
zur Herstellung eines Arzneimittels zur Ablösung von abnormem Keratinmaterial, wobei das abnorme Keratinmaterial aus der Gruppe Warzen, Schwielen, Hornhaut oder Fuß- und Fingernägel ausgewählt wird und wobei die Fuß- oder Fingernägel durch Pilzbefall oder Psoriaserkrankung verändert wurden.

14. Verwendung einer wässrigen Lösung, enthaltend Harnstoff in einer Menge von 15 % bis 35 %, bevorzugt von 25 % bis 33 %, und einen hydrophilen Filmbildner in einer Menge von 15 % bis etwa 35 %, bevorzugt von 17 % bis 25 %, jeweils bezogen auf das Gewicht der gesamten Lösung, zur Herstellung eines Arzneimittels zur Ablösung von abnormem Keratinmaterial.

15. Verwendung der Zubereitung nach einem oder mehreren der Ansprüche 1 bis 10, zur Hydratisierung von brüchigen Fuß- oder Fingernägeln

## Claims

1. A preparation which contains
a) urea in an amount from 41 percent by weight to 69 percent by weight, based on the nonvolatile constituents of the preparation,
b) a hydrophilic film-forming agent and
c) water or an alcohol-water mixture.

2. The preparation as claimed in claim 1, wherein urea is present in an amount from 41 percent by weight to 69 percent by weight, and/or the hydrophilic film-forming agent is present in an amount from 29% by weight to 59% by weight, in each case based on the nonvolatile constituents of the preparation.

3. The preparation as claimed in claim 2, wherein urea is present in an amount from 45% by weight to 65% by weight, preferably from 46% by weight to 63% by weight, in each case based on the nonvolatile constituents of the preparation.

4. The preparation as claimed in claim 3, wherein urea is present in an amount from 55% by weight to 63% by weight, in each case based on the nonvolatile constituents of the preparation.

5. The preparation as claimed in one or more of claims 1 to 4, wherein the hydrophilic film-forming agent employed is a compound from the group consisting of acrylic/methacrylic acid ester copolymers, polyvinylpyrrolidones, polyvinyl alcohols, vinyl acetate/vinylpyrrolidone copolymers, vinyl acetate/crotonic acid copolymers, methyl vinyl ether/maleic acid copolymers, polyesters, polyester amides, carboxymethylcellulose, hydroxyethylcellulose, hydroxypropylcellulose and hydroxypropylmethylcellulose or a mixture thereof.

6. The preparation as claimed in claim 5, wherein polyvinylpyrrolidones are employed as the hydrophilic film-forming agent.

7. The preparation as claimed in one or more of claims 1 to 6, wherein an alcohol from the group consisting of methanol, ethanol, propanol, isopropanol, butanol, pentanol und hexanol or a mixture thereof is employed in the aqueous-alcoholic solution.

8. The preparation as claimed in claim 7, wherein ethanol, n-propanol or isopropanol is employed as the alcohol.

9. The preparation as claimed in one or more of claims 1 to 8, wherein the ratio of alcohol to water is from 9 : 1 to 1 : 9; preferably 2 parts of alcohol to 3 parts of water are present.

10. The preparation as claimed in one or more of claims 1 to 9, wherein lactic acid is employed in an amount from 0.5% by weight to 5% by weight, based on the weight of the entire preparation.

11. The use of the preparation as claimed in one or more of claims 1 to 10, for the production of a pharmaceutical for the detachment of abnormal keratinous material.

12. The use as claimed in claim 11, wherein the abnormal keratinous material is selected from the group consisting of warts, calluses, hard skin or toe- and fingernails, where the toe- or fingernails have been changed by fungal attack or psoriatic disease.

13. The use of a preparation comprising
a) urea in an amount from 25 percent by weight to 35 percent by weight, based on the total weight of the preparation,
b) a hydrophilic film-forming agent in an amount from 15% by weight to 20% by weight, based on the total weight of the preparation, and
c) water or an alcohol-water mixture
for the production of a pharmaceutical for the detachment of abnormal keratinous material, where the abnormal keratinous material is selected from the group consisting of warts, calluses, hard skin or toe- and fingernails and where the toe- or fingernails have been changed by fungal attack or psoriatic disease.

14. The use of an aqueous solution comprising urea in an amount from 15% to 35%, preferably from 25% to 33%, and a hydrophilic film-forming agent in an amount from 15% to approximately 35%, preferably from 17% to 25%, in each case based on the weight of the entire solution, for the production of a pharmaceutical for the detachment of abnormal keratinous material.

15. The use of the preparation as claimed in one or more of claims 1 to 10, for the hydration of brittle toe- or fingernails.

## Revendications

1. Préparation, **caractérisée en ce qu'**elle contient
a) de l'urée en une quantité de 41 % en poids à 69 % en poids, par rapport aux composants non volatils de la préparation,
b) un agent filmogène hydrophile et
c) de l'eau ou un mélange d'alcool et d'eau.

2. Préparation selon la revendication 1, **caractérisée en ce que** l'urée est contenue en une quantité de 41 % en poids à 69 % en poids, et/ou l'agent filmogène hydrophile est contenu en une quantité de 29 % en poids à 59 % en poids, chaque fois par rapport aux composants non volatils de la préparation.

3. Préparation selon la revendication 2, **caractérisée en ce que** l'urée est contenue en une quantité de 45 % en poids à 65 % en poids, de préférence de 46 % en poids à 63 % en poids, chaque fois par rapport aux composants non volatils de la préparation.

4. Préparation selon la revendication 3, **caractérisée en ce que** l'urée est contenue en une quantité de 55 % en poids à 63 % en poids, chaque fois par rapport aux composants non volatils de la préparation.

5. Préparation selon une ou plusieurs des revendications 1 à 4, **caractérisée en ce qu'**on utilise en tant qu'agent filmogène hydrophile un composé choisi dans le groupe constitué par des copolymères ester d'acide acrylique/méthacrylique, des polyvinylpyrrolidones, des poly(alcool vinylique)s, des copolymères acétate de vinyle/vinylpyrrolidone, des copolymères acétate de vinyle/acide crotonique, des copolymères oxyde de méthyle et de vinyle/acide maléique, des polyesters, des polyesteramides, la carboxyméthylcellulose, l'hydroxyéthylcellulose, l'hydroxypropylcellulose et l'hydroxypropylméthylcellulose ou un mélange de ceux-ci.

6. Préparation selon la revendication 5, **caractérisée en ce que** des polyvinylpyrrolidones sont utilisées en tant qu'agent filmogène hydrophile.

7. Préparation selon une ou plusieurs des revendications 1 à 6, **caractérisée en ce que** dans la solution aqueuse-alcoolique on utilise un alcool choisi dans le groupe constitué par le méthanol, l'éthanol, le propanol, l'isopropanol, le butanol, le pentanol et l'hexanol ou des mélanges de ceux-ci.

8. Préparation selon la revendication 7, **caractérisée en ce que** l'éthanol, le n-propanol ou l'isopropanol est utilisé en tant qu'alcool.

9. Préparation selon une ou plusieurs des revendications 1 à 8, **caractérisée en ce que** le rapport de l'alcool à l'eau va de 9:1 à 1:9, de préférence 2 parties d'alcool sont contenues pour 3 parties d'eau.

10. Préparation selon une ou plusieurs des revendications 1 à 9, **caractérisée en ce qu'**on utilise de l'acide lactique en une quantité de 0,5 % en poids à 5 % en poids, par rapport au poids de la préparation totale.

11. Utilisation de la préparation selon une ou plusieurs des revendications 1 à 10, pour la fabrication d'un médicament destiné à l'élimination de matière kératinique anormale.

12. Utilisation selon la revendication 11, **caractérisée en ce que** la matière kératinique anormale est choisie dans le groupe constitué par les verrues, les callosités, la couche cornée et les ongles des mains et des pieds, les ongles des mains ou des pieds étant altérés par une attaque de champignons ou une maladie psoriasique.

13. Utilisation d'une préparation contenant
a) de l'urée en une quantité de 25 % en poids à 35 % en poids, par rapport au poids total de la préparation,
b) un agent filmogène hydrophile, lui en une quantité de 15 % en poids à 20 % en poids, par rapport au poids total de la préparation, et
c) de l'eau ou un mélange d'alcool et d'eau,
pour la fabrication d'un médicament destiné à l'élimination de matière kératinique anormale, la matière kératinique anormale étant choisie dans le groupe constitué par les verrues, les callosités, la couche cornée et les ongles des mains et des pieds, et les ongles des mains ou des pieds étant altérés par une attaque de champignons ou une maladie psoriasique.

14. Utilisation d'une solution aqueuse contenant de l'urée en une quantité de 15 % à 35 %, de préférence de 25 % à 33 %, et un agent filmogène hydrophile en une quantité de 15 % à environ 35 %, de préférence de 17 % à 25 %, chaque fois par rapport au poids total de la solution totale, pour la fabrication d'un médicament destiné à l'élimination de matière kératinique anormale.

15. Utilisation de la préparation selon une ou plusieurs des revendications 1 à 10, pour l'hydratation d'ongles cassants des mains ou des pieds.
